# EUROPÄISCHE PATENTSCHRIFT

(11) **EP 3 367 959 B1**
(45) Veröffentlichungstag und Bekanntmachung des Hinweises auf die Patenterteilung: **25.01.2023**
(21) Anmeldenummer: 16787444.5
(22) Anmeldetag: 26.10.2016
(51) Int. Cl.: A61C 8/00, A61C 9/00, A61K 6/84, A61K 6/844, A61K 6/887, A61K 6/891, A61K 6/90

(54) **SCANABUTMENT MIT VERGRÖSSERTER SCANFLÄCHE**
SCAN ABUTMENT WITH AN INCREASED SCANNING SURFACE
PILIER DE SCANNAGE POSSÉDANT UNE SURFACE DE SCANNAGE AGRANDIE

(30) Priorität: 27.10.2015 DE 102015118285
(43) Veröffentlichungstag der Anmeldung: 05.09.2018
(73) Patentinhaber: Kulzer GmbH, 63450 Hanau (DE)
(72) Erfinder: FUNK, Matthias, 50968 Köln (DE)
(74) Vertreter: Bendele, Tanja
(86) Internationale Anmeldenummer: PCT/EP2016/075795
(87) Internationale Veröffentlichungsnummer: WO 2017/072171

(56) Entgegenhaltungen:
- EP-A1- 2 829 250
- WO-A1-2015/040250
- CN-U- 204 484 369
- DE-A1-102008 028 214
- IT-A1- TO20 110 020
- KR-A- 20130 110 850
- KR-B1- 100 950 022
- US-A1- 2007 054 241
- US-A1- 2007 111 162
- US-B1- 6 168 436

## Beschreibung

Die Erfindung betrifft ein Scanabutment zur Bestimmung der Lage der Ebene der Stirnseite (koronal) eines dentalen enossalen Implantates, wobei das Abutment einen länglichen, hohlen Schaft und apikal einen Sockel an der Unterseite des Schaftes aufweist und der Sockel mit zumindest einem Teil seiner Unterseite (apikal) auf die Stirnseite (koronal) des Implantates anbringbar ist, wobei der Sockel eine von seiner Oberseite zur Unterseite des Sockels verlaufende Ausnehmung zur Aufnahme von Mitteln zur Festlegung des Abutments auf dem Implantat aufweist, wobei der längliche, hohle Schaft eine Seitenwand aufweist, die an ihrem äußeren Umfang mindestens eine Durchbrechung oder Vertiefung aufweist, insbesondere in Form eines Bogensegmentes, wobei vorzugsweise die Durchbrechung oder Vertiefung der Seitenwand von der Unterseite des Schaftes bis zur Oberseite des Schaftes reicht, wodurch mindestens eine planare Fläche in der Ebene zwischen Schaft und Sockel gebildet wird. Besonders bevorzugt ist die planare Fläche senkrecht zur Mittelachse des Implantates angeordnet. Vorzugsweise kann die planare Fläche alternativ oder zusätzlich parallel zur Ebene der Unterseite des Sockels und/oder der Ebene der Oberseite des Schaftes angeordnet sein.

WO2015040250A1 und ITTO20110020A1 offenbaren Abutments für Dentalimplantate. Abdruckpfosten für Dentalimplantate sind z.B. aus EP2829250A1, CN204484369U, und US2007/0054241A1 bekannt.

CIM-Prozesse (Computer-Integrated Manufacturing), wie CAD/CAM-Verfahren, gewinnen in der dentalen Prothetik zunehmend an Bedeutung. So ist es bei der Implantatprothetik inzwischen üblich, die Implantat- und Restzahnsituation im Patientenmund zu digitalisieren und den Zahnersatz über CAD/CAM zu gestalten und anzufertigen. Zur Digitalisierung der Ausrichtung der Implantate über das Scannen eines Gipsmodells oder auch der Implantate im Patientenmund kommen sogenannte Scanabutments zum Einsatz.

Scanabutments sind z.B. aus DE102008028214A1, KR20130110850A, und KR100950022B1 bekannt.

Scanabutments sind in der Regel zylinderförmige Vorrichtungen aus einem Kunststoff, die mit geringem Passungsspiel fest mit den Implantaten verschraubt werden. Diese Vorrichtungen erlauben es über einen optischen Scan mit einem Tisch- oder Intraoralscanner die dreidimensionale Lage der Implantate im Kiefer zu bestimmen. Der Scan der Scanabutments dient daher dazu die Lage der Implantate durch den Scan, einer parallel in der Höhe versetzen Ebene der Oberseite des Scanabutments und des Schaftes des Scanabutments, die Lage der Ebene der Stirnseite des Implantates sowie die Ausrichtung des Implantates möglichst genau zu vermessen. Dies funktioniert recht gut für Einzelimplantate, die mit einem Implantataufbau und einer Krone versorgt werden.

Die Scanabutments des Standes der Technik weisen dazu einen länglichen Schaft einer definierten Höhe auf, um die Achsrichtung und x-y Position des Implantates im Kiefer bestimmen zu können. Der Schaft ist in der Regel zylindrisch. In der einfachsten Ausführung sind die Schäfte kreisförmig, aber auch andere Querschnitte sind üblich. Des Weiteren sind z.B. auch kegelförmige, wobei der größere Durchmesser implantatseitig vorliegt und/oder kugelförmige Geometrien bekannt (z.B. CADstar).

Bei sogenannten Brückenkonstruktionen, bei denen zwei und mehr Implantate als Stütze für eine prothetische Versorgung, wie einer Dentalbrücke oder einen Steg, verwendet werden, reicht oft die Genauigkeit der Digitalisierung noch nicht aus, denn zur Herstellung passgenauer prothetischer Versorgungen über mehrere Implantate muss die okklusale Ebene der Implantate sehr genau ermittelt werden, da bei Brückenkonstruktionen die Implantate über die prothetische Versorgung miteinander in Verbindung stehen. Bei ungenauen Daten der okklusalen Ebene fehlt der angefertigten prothetischen Versorgung eine präzise Passung. Aufgrund der grossen Entfernung der gescannten Ebene am oberen Schaftende der Abutments in Bezug auf die koronale Ebene des Implanates wird die erforderliche Genauigkeit zur Bestimmung der Lage der Stirnseite bei bekannten Abutments nicht erzielt.

Aus diesem Grund müssen bislang bei der digitalisierten Herstellung der von Implantaten getragenen Dentalbrücken in der Produktion die Implantatpositionen anhand des Gipsmodells nachgescannt werden und die koronalen Auflageflächen (koronalen Ebenen der Implantate) trotz vorliegender 3D-Konstruktionsdaten neu bestimmt werden. Besonders aufwendig ist dieses Nachscannen, weil dazu das Gipsmodell zusammen mit den 3D-Konstruktiondaten vom Dentallabor zu einem Zentralfertiger eingesendet werden muss. Das Zentrallabor führt dann ein Nachscannen des Dentalmodells vor der Herstellung der von Implantaten getragenen Brücken durch und passt die digitalen Daten an.

Die Höhenbestimmung der Implantatstirnseiten der nachfolgend genannten Scanabutments erfolgt indirekt über die Bestimmung der Höhe und Ausrichtung der oberen Stirnseite der Scanabutments.

Der genannte Genauigkeitsverlust hat verschiedene Ursachen. Einerseits entsteht er durch die eingeschränkte Genauigkeit des Scanners und zum anderen durch die Toleranzen auf Seiten der Scanabutments. Bei den vorgenannten Brückenkonstruktionen kommt es insbesondere auch auf die genaue Bestimmung der Höhenlage der koronalen Implantatstirnseite(n) an. Abweichungen dieser Höhenpositionen führen unweigerlich zu einer nicht passgenau anbringbaren Brücke, im schlimmsten Fall hat die Brücke eine Bewegungsfreiheit, weil die Auflageflächen der Implantatbrücken und der Implantatstirnseiten zueinander im 3D-Datensatz nicht passgenau waren.

Eine Aufgabe der Erfindung bestand darin, Scanabutments zu entwickeln, die die genannten Nachteile bezüglich der Ungenauigkeiten beim Einscannen vermindern und bestenfalls ein Nachscannen vermeiden. Insbesondere bestand die Aufgabe Scanabutments zu entwickeln, die eine verbesserte Datengenauigkeit der mesialen, bukkalen, labialen, oralen und/oder okklusalen Ebenen der/des Scanabutments und damit der koronalen Stirnseite(n) und der Lage des/der Implantat(e)s ermöglichen.

Die Erfindung gibt nun einen Lösungsvorschlag, wie die Toleranzen dieser Höhenposition innerhalb der Scanabutments möglichst gering gehalten werden können, nämlich indem der Abstand der planparallelen Ebene der koronalen Stirnseite des Implantates und der zu scannenden Fläche(n) des Scanabutments deutlich vermindert wird. Gelöst wird die Aufgabe mit einem Scanabutment gemäß Anspruch 1, wobei vorteilhafte Ausgestaltungen in den Unteransprüchen sowie in der Beschreibung detailliert dargestellt sind.

Bekannte Scanabutments bestimmen diese Höhenlage der jeweiligen Implantatstirne(n) durch einen Scan der oberen Stirnseite des auf dem jeweiligen Implantat angebrachten Abutments. Die koronale Stirnseite der Scanabutments ist in der Regel jedoch etwa 10 mm und mehr von der Stirnseite der Implantate entfernt. Entsprechend hoch sind dadurch die Fertigungstoleranzen dieser Geometrie für bekannte Scanabutments, da sich das Maß über die gesamte Länge des Scanabutments erstreckt, wobei gilt, "je länger ein Maß ist, umso schwieriger ist es eine kleine Toleranz einzuhalten".

Kern der Erfindung ist es, den Abstand und damit die Toleranzen des Scanabutments und des Scans zwischen Implantatstirnseite und der Fläche des Scanabutments, der für die Höhenlage beim Scannen erfasst wird, möglichst gering zu halten (Maß "X" in Zeichnung).

Gegenstand der Erfindung ist eine Scanabutment nach Anspruch 1.

Erfindungsgemäß wird eine Bezugskante, vorliegend Fläche(n) 6 und 7 genannt, bereitgestellt, die nicht mehr auf der koronalen Stirnfläche des Scanabutments synonym zur Oberseite des Schaftes des Scanabutments in einem Abstand von etwa 10 bis 15 mm zur koronalen Stirnseite des Implantates liegt, sondern bevorzugt nur in einem Abstand von nur noch 1 bis 3 mm. Auf diese Weise können die Toleranzen um ca. 50% vermindert werden. Diese Verbesserung der Toleranz entspricht einer Verbesserung der Toleranz von etwa 20 bis 30 µm (Mikrometer), so dass eine erhebliche Verbesserung der Scangenauigkeit erzielt wird. So gibt die DIN ISO 2768-1 für Allgemeintoleranzen - fein von 2 +/-0,05 mm bis zu 10 +/-0,1 mm an. Die ISO 286 nennt ein Toleranzsystem für Längenmaße von Kunststoffdrehteilen der Kategorie A gemäß Toleranzreihe 9 und Maßen von 1 bis 3 mm von 0,025 mm und für Maße von 10 bis 18 mm von 0,043 mm.

Das erfindungsgemässe Scanabutment ist werkstoffeinstückig, d.h. ein integrales Scanabutment.

Aufgrund der geringen Durchmesser der Implantate, die in der Regel zwischen 3,3 und 6,0 mm betragen, wird jedoch der gesamte zur Verfügung stehende Durchmesser für den Schaft des Scanabutments benötigt. Folglich fiel automatisch die gesamte Fläche für die Höhenbestimmung auf die obere Stirnseite des Scanabutments, synonym Oberseite des Schaftes. Die obere Stirnseite des Abutments ist jedoch am weitesten von der Stirnseite des Implantates entfernt. Um nun eine parallele Fläche zur Stirnseite des Implantates mit kleinstmöglichem Abstand, z.B. 2 mm, zu bekommen, wird erfindungsgemäss vorgeschlagen den Schaft, vorzugsweise in einer Alternative auf 90° seines Umfanges, zu unterbrechen. Diese Durchbrechung des Schaftes kann größer oder kleiner als 90° ausgebildet sein, insbesondere in Form eines Bogensegmentes. Sofern eine Durchbrechung mit einem Öffnungswinkel von kleiner < 90° gewählt wird, weist die gebildete Fläche nicht die optimalste Größe auf und es kann vorkommen, dass die Fläche nicht mit der gewünschten Genauigkeit erfasst werden kann. Wird ein Öffnungswinkel der Durchbrechung von größer > 120° gewählt, sinkt die Scangenauigkeit des Schaftes. Eine Balance und Steigerung der Scangenauigkeit der parallelen Fläche die durch die Durchbrechung gebildet wird und der Oberseite des Schaftes des Scanabutments wird bei einem Öffnungswinkel von etwa 90° bis kleiner gleich 120° erzielt (je +/- 10°, insbesondere +/- 5°).

Bei Scanabutments für größere Implantatdurchmesser ab etwa 4,5 mm kann alternativ oder zusätzlich eine Abstufung des unteren Bereiches des Schaftes zum Sockel erfolgen, wodurch eine weitere Fläche, insbesondere eine umlaufende weitere Fläche, entsteht. Diese Fläche kann der Vergrößerung der planparallelen Gesamtfläche (Flächen 6+7) dienen, die wiederum vorzugsweise planparallel zur koronalen Implantatstirnseite angeordnet ist. Diese vergrößerte Fläche kann noch besser und genauer vom Scanner erfasst werden. In allen erfindungsgemäßen Ausführungsformen wird vorzugsweise im Scanvorgang zusätzlich die verbleibende Oberseite des Schaftes eingescannt, die vorzugsweise ebenfalls parallel zu den erfindungsgemäßen Flächen des Scanabutments angeordnet ist.

Um die Vorteile dieser Geometrie auszunutzen, wurden der Scanner und der Algorithmus der dazugehörigen Software zur Erfassung der neuen mindestens einen Fläche angepasst, so dass sie beim Ausrichten des Scans mit den CAD-Scanabutmentbibliotheken die mindestens eine neue Fläche berücksichtigen, die der Implantatstirnseite am nächsten ist. Für den Scann der erfindungsgemässen Scanabutments wird die Software 3shape verwendet, wobei auch andere Systeme entsprechend angepasst werden können.

Gegenstand der Erfindung ist somit ein Scanabutment zur Bestimmung der Lage (x,y,z-Koordinaten) der Stirnseite eines dentalen enossalen Implantates, insbesondere im Kiefer eines Patienten oder als Laboranalog in einem Modell, wobei das Abutment einen länglichen, hohlen Schaft und einen Sockel an der Unterseite des Schaftes aufweist und der Sockel mit zumindest einem Teil seiner Unterseite auf die Stirnseite des Implantates anbringbar ist, wobei der Sockel eine von seiner Oberseite zur Unterseite des Sockels verlaufende Ausnehmung aufweist, insbesondere zur Aufnahme von Mitteln zur Festlegung des Abutments auf dem Implantat, wobei der längliche, hohle Schaft aus einer Seitenwand gebildet wird, und die Seitenwand zumindest teilweise im Bereich unmittelbar oberhalb des Sockels an ihrem äußeren Umfang mindestens eine Durchbrechung und/oder Vertiefung in der Seitenwand, insbesondere in Richtung auf die Längsachse des Schaftes, aufweist, wodurch mindestens eine planare Fläche durch die Durchbrechung und/oder die Vertiefung in der Ebene zwischen Schaft und Sockel gebildet wird, wobei sich die planare Fläche auf der Oberseite des Sockels bildet. Vorzugsweise kann die Fläche senkrecht zur Mittelachse des Implantates angeordnet werden. Des Weiteren ist es besonders bevorzugt, wenn die Mittelachse des Scanabutments und die Mittelachse des Implantates koaxial angeordnet werden können. Weitere Durchbrechungen können zu planaren Flächen führen, die parallel zur ersten Fläche verschoben und/oder horizontal auf dem Umfang der Seitenwand liegen können. Ebenso kann es bevorzugt sein, wenn die Flächen alternativ parallel zur Ebene der Unterseite des Sockels und/oder der Ebene der Oberseite des Schaftes angeordnet sind.

Als Durchbrechung gilt eine durchgehende Ausnehmung, also ein Loch, in der Seitenwand, die von der äusseren Oberfläche der Seitenwand bis zur inneren Oberfläche der Seitenwand des hohlen Schaftes reicht. Eine Vertiefung in der Seitenwand des Schaftes umfasst keinen Schaft in Form eines Kegelschnittes mit planarer Seitenwand. Eine Vertiefung umfasst mindestens eine Hinterschneidung in der Seitenwand des Schaftes, die von der äußeren Oberfläche des Schaftes in die Seitenwand hineinreicht aber nicht bis zur inneren Oberfläche der Seitenwand ausgebildet ist.

Vorzugsweise kann die Unterseite des Sockels passgenau auf der Stirnseite des Implantates angebracht werden. Dazu weist der Sockel insbesondere eine Anschlussgeometrie auf, die passend zu der Anschlussgeometrie des jeweils vorliegenden Implantates ausgebildet ist. Die Mittel zur Festlegung des Abutments auf dem Implantat können umfassen Schrauben, Nägel, Bolzen oder weitere dem Fachmann hinlänglich bekannte Mittel zur Befestigung von Abutments auf Implantaten.

Unter den erfindungsgemäßen jeweiligen Ebenen werden jene geometrischen Ebenen verstanden, die sich bspw. in die jeweiligen Oberflächen, Stirnseiten, Oberseiten oder Unterseiten legen lassen, wie insbesondere die Ebene zwischen Schaft und Sockel, so wie sich jeweils eine Ebene in der Oberfläche des Schaftes oder zwischen der Unterseite des Schaftes und der Oberseite des Sockels oder in die Stirnseite des Implantates legen lässt.

Der längliche, hohle Schaft wird gebildet durch die Seitenwand, die die äussere Begrenzung des Schaftes bildet, wobei der hohle Schaft ein innen, entlang der Längsachse verlaufendes Lumen aufweist. Die Seitenwand ist insbesondere zylindrisch, und kann erfindungsgemäss durchbrochen sein und/oder einen geringeren Durchmesser als der Sockel aufweisen.

Erfindungsgemäß kann der längliche Schaft weitere horizontale und/oder vertikale, planare Durchbrechungen und/oder Vertiefungen aufweisen, die alle vorzugsweise planparallel zu der mindestens einen Fläche und optional der weiteren Fläche ausgebildet werden. Nach einer weiteren Alternative kann der Schaft vertikale, lineare Durchbrechungen und/oder Vertiefungen oder auch vertikale, planare Flächen aufweisen, um die Scangenauigkeit in der Trajektorie der Implantatstirnseite weiter zu verbessern.

Ferner weist der Schaft (ohne Sockel) eine definierte Höhe (Y) von 5 mm bis 20 mm auf, insbesondere von 5 bis 15 mm, bevorzugt um 8 bis 13 mm, insbesondere mit einer Toleranz von plus/minus 0,05 mm, vorzugsweise bis plus/minus 0,02 mm. Somit werden trotz einer geringen Gesamthöhe (Schafthöhe Y und Sockelhöhe X) von 9 mm bis 15 mm, die Scandaten um etwa 20 bis 50 % bezüglich der Fehlertoleranz verbessert. Dieser Verbesserung entspricht einer Höheren Genauigkeit von etwa 20-30 µm (Mikrometer)

Die Höhe (X) des Sockels liegt erfindungsgemäß vorzugsweise bei größer gleich 0,1 bis 10 mm, insbesondere von 0,75 mm bis 5 mm, bevorzugt um 1 bis 3 mm, insbesondere mit einer Toleranz von plus/minus 0,1 mm, bevorzugt bis plus/minus 0,02 mm, vorzugsweise bis plus/minus 0,005 mm.

Ein bevorzugtes Verhältnis der Höhe X zur Höhe Y liegt bei 1 : 1,5 bis 1: 20. Bevorzugt liegt das Verhältnis im Bereich von 1 : 5 bis 1 : 13.

Nach einer weiteren bevorzugten Ausgestaltung der Erfindung weist die Seitenwand des länglichen, hohlen Schaftes zumindest teilweise entlang einer Längsseite des Schaftes mindestens eine Durchbrechung auf. Insbesondere beträgt die mindestens eine Durchbrechung von 5% bis 100% der Höhe einer Längsseite des Schaftes, vorzugsweise von 10 % bis 100%, bevorzugt 10% bis 90%, 20% bis 80% oder 20 bis 70%. Bevorzugt können auch entlang einer Längsseite größer gleich zwei Durchbrechungen in der Seitenwand angeordnet sein, insbesondere von 2 bis 10, 2 bis 5 oder 2 bis 3, die dann vertikal oder versetzt übereinander angeordnet sein können. Alternativ können am äußeren Umfang der Seitenwand mindestens zwei voneinander horizontal beabstandete Durchbrechungen vorgesehen sein, die jeweils zumindest teilweise entlang einer Längsseite des Schaftes verlaufen, die horizontal beabstandeten Durchbrechungen können beispielsweise in einem Winkel von 2° bis 330°, wie bspw. von 5° bis 90°, voneinander beabstandet sein. Jede Durchbrechungen weist vorzugsweise jeweils unabhängig eine Höhe von mindestens 10 % bis 100% der Höhe des Schaftes auf, vorzugsweise 15% bis 100%, 20 bis 80% oder 10% bis 30%. Bevorzugt sind entlang einer Längsseite größer gleich zwei bis 10 Durchbrechungen in der Seitenwand angeordnet, insbesondere 2 bis 10, 2 bis 5, 2 bis 3, die vorzugsweise jeweils 10 % bis 30% der Höhe einer Längsseite des Schaftes betragen.

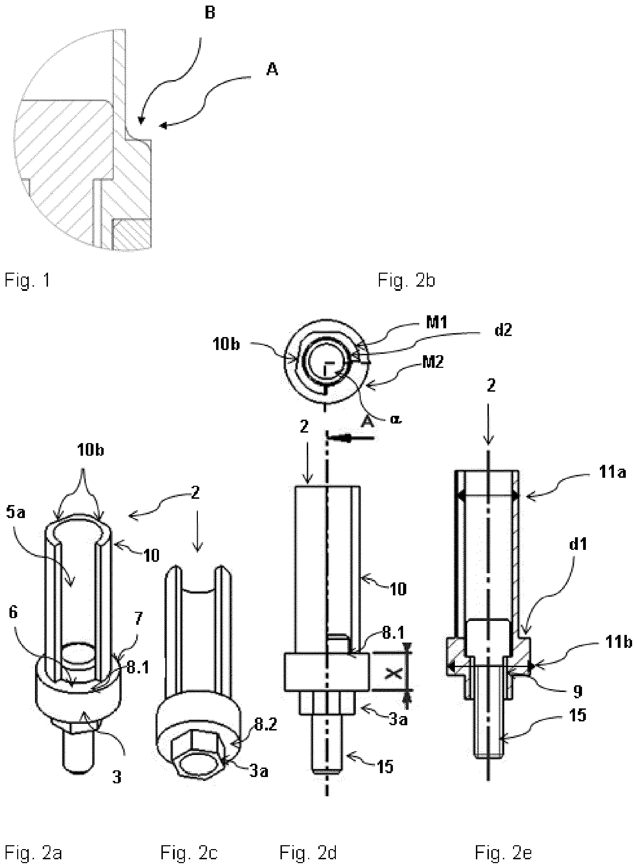

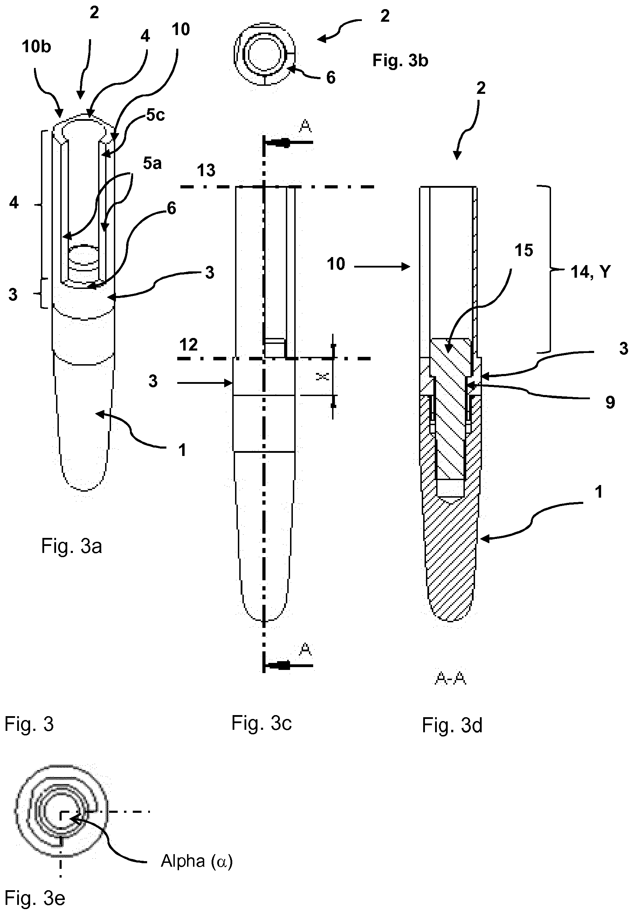

Bevorzugt ist eine Durchbrechung in Form eines Bogensegmentes mit einem Öffnungswinkel (alpha, α) von 80° bis 130°, vorzugsweise von etwa 90° bis 120°, wie in den Figuren 2 und 3 dargestellt. Nach weiteren Alternativen weist die Seitenwand eine Vielzahl an vertikal in der Höhe planparallel zueinander angeordneten Flächen auf, insbesondere in Form von vertikal, planparallel übereinander angeordneten Bogensegmenten, die vorzugsweise einen vorgenannten Öffnungswinkel aufweisen.

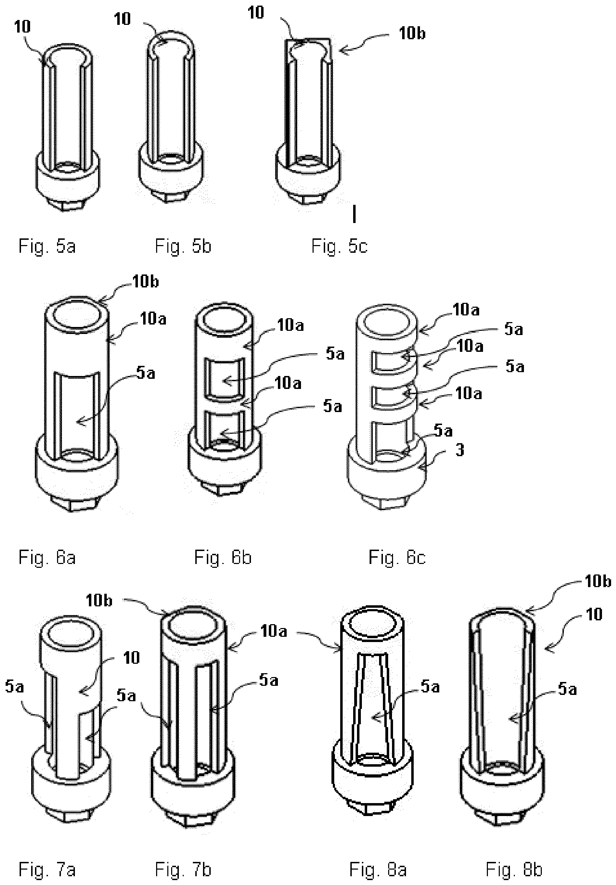

Somit kann die Seitenwand des Schaftes nach alternativen Ausführungsformen eine Vielzahl an Durchbrechungen aufweisen. Dabei können die Durchbrechungen auch rechteckige oder quadratische Ausnehmungen darstellen (Fig. 5a, 5b). Zur maximalen Steigerung der Scanauflösung können eine Vielzahl an Durchbrechungen bspw. horizontale, lammellenartige Durchbrechungen oder vertikale, lammellenartige Durchbrechungen in der Seitenwand ausgebildet sein, so dass die Seitenwand des Schaftes bspw. gitterförmig ausgebildet ist.

Ferner umfasst der Gegenstand der Erfindung ein Scanabutment, dessen Seitenwand des länglichen, hohlen Schaftes an einer Längsseite des Schaftes mindestens eine Durchbrechung mit einem Öffnungswinkel von 5° bis 330° aufweist, insbesondere im äußeren Umfang der Seitenwand, gemessen von einer Längsachse des Schaftes. Vorzugsweise weist die Seitenwand eine Durchbrechung mit einem Öffnungswinkel größer gleich 10 bis 120° auf, insbesondere von 30° bis 120°, vorzugsweise von 90° von plus/minus 20°, bevorzugt von plus/minus 10°, plus/minus 5°. Nach besonders bevorzugten Alternativen weist die die Seitenwand mindestens eine Durchbrechung mit einem Öffnungswinkel größer gleich 90° bis 120°, vorzugsweise von 100° mit plus/minus 15°, bevorzugt plus/minus 10°, besonders bevorzugt von plus/minus 5° auf. Entsprechend einer weiteren bevorzugten Alternative weist die Seitenwand des länglichen, hohlen Schaftes an einer Längsseite des Schaftes mindestens eine Durchbrechung mit einem Öffnungswinkel von 90° auf, insbesondere mit plus/minus 30°, wobei der Öffnungswinkel der Durchbrechung ausgehend von der Längsachse in Richtung des äußeren Umfangs der Seitenwand gemessen wird. Die Durchbrechung kann sich über die gesamte Länge des Schaftes oder einen Teil des Schaftes erstrecken.

Nach einer weiteren Ausgestaltung der Erfindung reicht die mindestens eine Durchbrechung der Seitenwand und/oder die Vertiefung am äußeren Umfang der Seitenwand des Schaftes von der Unterseite des Schaftes bis zur Oberseite des Schaftes.

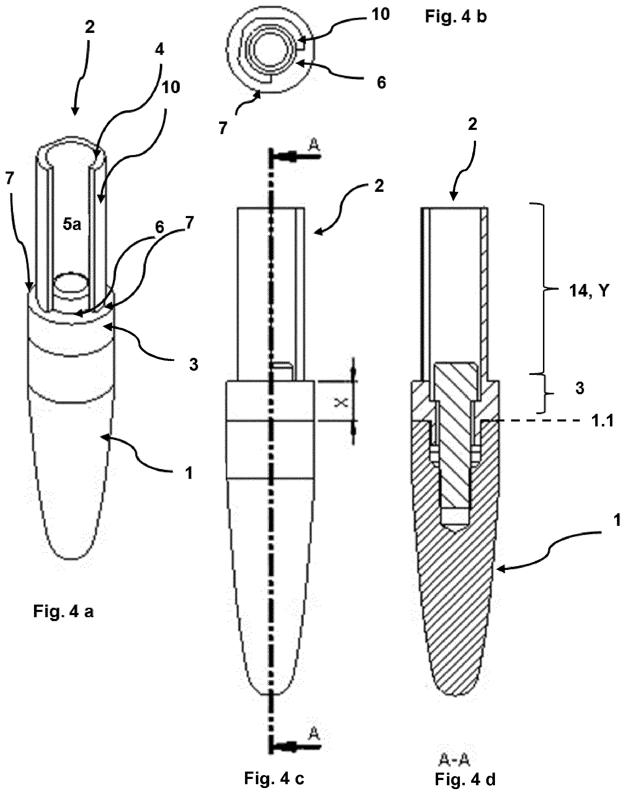

Ferner können die folgenden Alternativen realisiert sein, in denen die Seitenwand des Schaftes an ihrem äußeren Umfang
a) mindestens eine Durchbrechung in Form eines Bogensegmentes, Kreissegmentes, Segmentes einer Ellipse oder eines Dreiecks, insbesondere in der Ebene eines Querschnitts des Scanabutments, d.h. in der Draufsicht ausgehend von einer Längsachse als Bezugspunkt aufweist, wie in den Figuren 3a, 3b und 4b dargestellt, , oder
b) mindestens eine Durchbrechung im äußeren Umfang der Seitenwand in Form eines Vierecks aufweist, wie Rechtecks, Quadrates, oder Trapezes, wie in Figuren 8a und 8b dargestellt, insbesondere reicht die mindestens eine Durchbrechung in a) und/oder b) in der Seitenwand des Schaftes von der Unterseite des Schaftes bis zur Oberseite des Schaftes, vorzugsweise entspricht die Höhe der Durchbrechung mindestens 10% der Höhe des Schaftes, oder
c) mindestens eine Vertiefung in Form eines Bogensegmentes, Kreissegmentes, Segmentes einer Ellipse oder in Form einer U- oder V-förmigen Nut, U- oder V-förmigen Rinne, wie in den Figuren 6a und 6b, oder in Form eines Hohlzylinders aufweist, insbesondere wird dadurch die mindestens eine Fläche in einer vorgenannten Form gebildet. Die Vertiefung kann vorzugsweise vorliegen als eine Nut oder Rinne, die begrenzt wird von mindestens zwei Seitenwänden und einer dazwischenliegenden Wand mit vorzugsweiser rechtwinkligen U-förmigen Anordnung. Alternativ können zwei Seitenwände im spitzen Winkel V-förmig vorliegen.

Gemäß der Erfindung weist ein Scanabutment einen Schaft auf, bei dem mindestens ein äußerer Durchmesser des länglichen Schaftes kleiner als einer der äusseren Durchmesser des Sockels ist, wobei mindestens eine weitere planare Fläche in der Ebene zwischen Schaft und Sockel gebildet wird, so dass sich eine planare Gesamtfläche als Summe aus der einen planaren Fläche und der weiteren planaren Fläche bildet. Bevorzugt ist die planare Fläche in einem rechten Winkel zur Achse des Implantates angeordnet. Die weitere planare Fläche ist planparallel zur mindestens einen Fläche. Der äussere Durchmesser des länglichen Schaftes ist kleiner als der äussere Durchmesser des Sockels, wobei sich mindestens eine weitere planare Fläche in der Ebene zwischen Schaft und Sockel bildet, die Teil einer planaren Gesamtfläche in einer Ebene als Summe aus der einen planaren Fläche und der weiteren planaren Fläche ist.

Die erfindungsgemäßen Scanabutments weisen mindestens eine Fläche(n) und mindesten eine weitere Fläche auf, die zueinander planparallel in einer Ebene angeordnet sind. Vorzugsweise ist die Seitenwand des Schaftes in ein, zwei, drei bis acht Bereichen abgeflacht, vorzugsweise ist die Seitenwand planar ausgebildet. Vorzugsweise ist die Seitenwand in diesen Bereichen von der Oberseite des Sockels bis zur Oberseite des Schaftes planar ausgebildet.

Insbesondere bei Scanabutments mit größerem Durchmesser, ist der mindestens eine äußere Durchmesser des länglichen Schaftes kleiner als der äußere Durchmesser des Sockels oder einer der äußeren Durchmesser des Sockels, wobei mindestens eine weitere planare, vorzugsweise um den Schaft umlaufende Fläche in der Ebene zwischen Schaft und Sockel gebildet wird. Dies kann beispielsweise bei elliptischen oder rechteckigen Schaftgeometrien der Fall sein. Vorzugsweise ist diese Fläche senkrecht zur Mittelachse des Implantates angeordnet. In Alternativen kann sie planparallel zur Ebene der Unterseite des Sockels und/oder der Ebene der Oberseite des Schaftes und insbesondere planparallel zur mindestens einen Fläche angeordnet sein.

Nach einer weiteren Alternative kann die Dicke (d₂) der Seitenwand des Schaftes am äußeren Umfang des Schaftes variieren, insbesondere weist der äußere Umfang der Seitenwand mindestens zwei Durchmesser auf, wie beispielsweise bei Ellipsen. Bei einem elliptischen äußeren Umfang des Schaftes entlang der Längsachse können beispielsweise zwei vergrößerte weitere Flächen gebildet werden. Besonders bevorzugt ist daher auch eine Ausführungsform in der der äußere Umfang der Seitenwand des hohlen Schaftes entlang der Längsachse zumindest teilweise elliptisch ist, wie beispielweise bei einem Schaft in Form eines elliptischen Zylinders.

Als ein äusserer Durchmesser des länglichen Schaftes gilt jeder Durchmesser des länglichen Schaftes. Bevorzugt wird als ein äusserer Durchmesser des Schaftes der größte äussere Durchmesser des länglichen Schaftes verstanden.

Nach einer weiteren Ausgestaltung der Erfindung bilden die mindestens eine planare Fläche und die eine weitere planare Fläche die Oberfläche des Sockels zur Verbesserung der Scangenauigkeit. Beide Flächen sind bevorzugt senkrecht bzw. in der Normalen zur Mittelachse des Implantates angeordnet. Dabei sind vorzugsweise zugleich die Achse des Scanabutments und des Implantates koaxial angeordnet.

Bei bevorzugten Scanabutments kann die Seitenwand des Schaftes die Form eines a) Zylinders, insbesondere eines Hohlzylinders, vorzugsweise eines Kreiszylinders, eines elliptischen Zylinders, Prismas, wie beispielsweise mit rechteckiger oder quadratischer, wie in Figur 5c dargestellt, Ober- und Unterseite aufweisen bevorzugt mit rechteckigem oder quadratischem Querschnitt, und vorzugsweise mit abgerundeten vertikalen Kanten, bevorzugt ein gerades Prisma mit nahezu rechteckigem Querschnitt mit abgerundeten Kanten; oder b) die Seitenwand die Form eines Kegels oder c) Ellipsoids (Fig. 5b), d) Polyeders, insbesondere eines regulären und konvexen Polyeders, vorzugsweise eines Oktaeders oder Ikosaeders, oder einer e) Kugel aufweisen, wobei in a), b) oder c) die Seitenwand des Schaftes am äußeren Umfang mindestens eine Durchbrechung und/oder Vertiefung aufweist, insbesondere entlang einer Längsachse des Schaftes, wodurch mindestens eine planare Fläche durch die Durchbrechung und/oder die Vertiefung in der Ebene zwischen Schaft und Sockels gebildet wird, insbesondere bildet sich die planare Fläche auf der Oberseite des Sockels. Vorzugsweise kann die Fläche parallel zur Ebene der Unterseite des Sockels und/oder der Ebene der Oberseite des Schaftes angeordnet sein. Nach weiteren bevorzugten Alternativen kann die äußere Oberfläche der Seitenwand des Scanabutments zudem mindestens teilweise abgeflacht bzw. planar ausgebildet sein.

Bei besonders bevorzugten Scanabutments kann die Seitenwand des Schaftes die Form eines Zylinders, insbesondere eines gerades Prisma aufweisen, vorzugsweise mit der Grundfläche eines Vier- oder Fünfecks mit einer Kantenlänge der Grundfläche von 3,5 mm bis 6,5 mm, besonders bevorzugt mit nahezu rechteckigem Querschnitt. Dabei ist es besonders bevorzugt, wenn das gerade Prisma, insbesondere mit rechteckige, bevorzugt mit quadratischem Querschnitt, abgerundete vertikale Kanten in der Höhe des Prismas aufweist. Der Radius der Kanten beträgt vorzugsweise von 0,1 bis 1 mm.

Nach einer weiteren bevorzugten Alternative kann der Sockel des Scanabutments in Form eines Kegelstumpfes vorliegen, wobei vorzugsweise die Grundfläche des Kegelstumpfes die planare Oberseite des Sockels bildet und insbesondere die Deckfläche des Kegelstumpfes die Unterseite des Sockels bildet. Zweckmässig kann die planare Deckfläche die planare Oberseite des Sockels bilden.

In besonders bevorzugten Ausgestaltungen der Erfindung kann die Tiefe (= Bogenradius, r_{B}) der Fläche in der Ebene gemessen vom äußeren Umfang (M₁ = Mantel) der Seitenwand bis zur Längsachse des Schaftes von größer gleich 0,2 bis 4 mm betragen, insbesondere von 0,25 bis 0,8 mm, vorzugsweise von 0,3 bis 0,8 mm, besonders bevorzugt von 0,4 bis 0,7 mm.

Nach einer weiteren besonders bevorzugten Ausgestaltung der Erfindung kann die mindestens eine Tiefe (d₁) der weiteren Fläche in der Ebene gemessen vom äußeren Umfang (M₂) des Sockels bis zum äußeren Umfang (M₁) des Schaftes von größer gleich 0,2 bis 4 mm betragen, insbesondere von 0,25 bis 1,5 mm, vorzugsweise von 0,3 bis 0,8 mm, besonders bevorzugt von 0,4 bis 0,7 mm. Figuren 5 bis 10 stellen eine solche Ausgestaltung dar.

Die erfindungsgemäßen Scanabutments weisen vorzugswiese als die mindesten eine Fläche(n) 6 und mindesten eine weitere Fläche 7, die zueinander planparallel angeordnet sind, in der Summe eine Fläche(n) mindestens von 1 mm² bis 25 mm² auf, vorzugweise von 5 bis 20 mm²., besonders bevorzugt von 7 bis 15 mm², alternativ bevorzugt sind 7 bis 20 mm² oder 7 bis 25 mm².

Des Weiteren kann es bevorzugt sein, dass die Oberflächen des Scanabutments zumindest teilweise mit einer rauen Oberflächenstruktur versehen sind, vorzugsweise können die mindestens eine Fläche und die weitere Fläche sowie optional die äußere Fläche der Seitenwand und optional die Oberseite des Schaftes mit einer rauen Oberflächenstruktur versehen sein. Zur Veränderung der Oberfläche bieten sich an Pulverbeschichtung, Sandstrahlen, Ätzen etc., ohne die Verfahren auf diese Verfahren beschränken zu wollen.

Das Material der erfindungsgemäßen Scanabutments kann ausgewählt sein aus einem polymeren Material, gefülltem polymeren Material, Metall, einer Legierung, wie Titan, Kobaltchrom, Gold, einem faserverstärktem Polymer, einer Legierung, Hybridmaterial.

Dabei kann das polymere Material ausgewählt sein aus Fluorpolymeren, Polysulfid enthaltenden Polymeren, Polysulfon enthaltenden Polymeren, Polyaryletherketonen, Polyimiden, PC (Polycarbonat), PAEK (Polyaryletherketonen), PEEK (Polyetheretherketonen), PEK (Polyether-ketonen); PEKK (Poly(etherketonketonen)), PEEEK (Poly(etheretheretherketonen)), PEEKK (Poly(etheretherketonketonen)), PEKEKK (Poly(etherketon-etherketon-ketonen)); PES (Polyarylsulfonen), PPSU (Polyarylsulfonen), PSU (Polysulfonen), PPS (Polyphenylensulfiden), PFA (Perfluoralkoxy-Polymeren), PFE (Poly(fluorenylen ethynylen)polymeren); PVDF (Polyvinylidenfluoriden), PCTFE (Polytetrafluoroethylen), PAI (Polyamideimiden); PI (Polyimiden), PEI (Polyetherimiden), PBI (Polybenz-imidazolen). Als metallische Legierungen oder Metalle sind Kobaltchrom, Titan, Gold etc. bevorzugt.

Offenbart wird auch die Verwendung eines Scanabutments wobei die eine Fläche und/oder die mindestens eine weitere Fläche a) senkrecht zur Mittelachse des Implantates angeordnet sind, wenn das Scanabutment zur Bestimmung der Lage (x,y,z-Koordinaten) der Stirnseite eines dentalen enossalen Implantates mit seiner Unterseite Sockels auf der Stirnseite des Implantates angebracht ist, oder b) die Mittelachse des Scanabutments koaxial zur Mittelachse des Implantates angeordnet ist und die eine planare Fläche und/oder die mindestens eine weitere Fläche senkrecht zur Mittelachse des Implantates angeordnet ist.

Die erfindungsgemässen Durchbrechungen und/oder Vertiefungen, die die erste Fläche bilden und optional die weiteren Flächen können unabhängig von den vorstehenden Ausführungen für alle dem Fachmann sinnvoll erscheinenden Schaftgeometrien und Materialien unabhängig angewandt werden.

Die Erfindung wird anhand der Figuren näher erläutert, ohne die Erfindung auf diese Ausführungsbeispiele zu begrenzen.

Die Figuren stellen dar: **Fig. 1****:** Erfassung von kleinen Flächen A durch einen Scanner werden abgerundet entsprechend B vom Scanner erfasst; **Fig. 2a****-e:** Erfindungsgemässes Scanabutment ohne Implantat mit Implantatschraube für mittlere bis große Durchmesser bspw. 4,5 mm, **Fig. 2a****:** Perspektivische Ansicht des Scanabutments **2** mit Implantat **1,** **Fig. 2b****:** Axialer Querschnitt des Scanabutments **2,** **Fig. 2c****:** Ansicht des Scanabutments von schräg unten, **Fig. 2d****:** Seitliche Ansicht der Ebene A, **Fig. 2e****:** Longitudinaler Querschnitt; **Fig. 3a****-e:** Ein Scanabutment **2** für kleine Durchmesser bspw. 3,3 mm, welches nicht unter den Schutzbereich der Ansprüche fällt; **Fig. 3a****:** Perspektivische Ansicht des Scanabutments **2** mit Implantat **1,** **Fig. 3b****:** Axialer Querschnitt des Scanabutments **2,** **Fig. 3c****:** Seitliche Ansicht der Ebene A, **Fig. 3d****:** longitudinaler Querschnitt; **Fig. 3e****:** Öffnungswinkel alpha (a) 90° der Durchbrechung, **Fig. 4a****-d:** Erfindungsgemäßes Scanabutment für mittlere bis große Durchmesser bspw. 4,5 mm, **Fig. 4a****:** Perspektivische Ansicht des Scanabutments **2** mit Implantat **1,** **Fig. 4b****:** Axialer Querschnitt des Scanabutments **2,** **Fig. 4c****:** Seitliche Ansicht der Ebene A, **Fig. 4d****:** longitudinaler Querschnitt, **Fig. 5a****-c:** Alternative Ausführungsformen des Scanabutments mit kreisrundem äusseren Schaft (**Fig. 5a**), mit elliptischem äusseren Schaft (**Fig. 5b**), mit viereckigem äusseren Schaft (**Fig. 5c**); **Fig. 6a****-c:** Alternative Scanabutments mit Durchbrechungen **5a** entlang einer Längsachse des Schaftes (**Fig. 6a** und **6b****:** Öffnungswinkel alpha = 90°); **Fig. 7a** und **7b****:** Scanabutment mit zwei horizontal beabstandeten Durchbrechungen **5a;** **Fig. 8a** und **8b****:** Scanabutments mit einer Durchbrechung **5a** der Seitenwand am äusseren Umfang; **Fig. 9a** und **9b****:** Scanabutment **2** mit U-förmigen Vertiefungen **5b;** **Fig. 10a** und **10b****:** Scanabutment **2** mit einem kegelstumpfartigen Sockel **3.**

**Figur 1** zeigt die mit abgerundeten Kanten A durch einen Scanner erfasste zu kleine Fläche B. Die **Figuren 2a** bis **2e** stellen ein erfindungsgemässes Scanabutment **2** ohne Implantat **1** mit Befestigungsmittel **15** dar. Das Scanabutment **2** der **Figur 2a** weist eine Durchbrechung **5a** in der Seitenwand **10** mit einem Öffnungswinkel **α** von **90° (****Fig. 2b****)** auf. Die äußere Oberfläche der Seitenwand **10** des Scanabutments **2** kann zudem abgeflacht bzw. planar **10b** sein, so dass sich weitere planare Flächen auf der äusseren Oberfläche des Schaftes **4** ausbilden. **Fig. 2a****-c** zeigen die planare Oberseite **8.1** und die planare Unterseite **8.2** des Sockels sowie die 6-Kantanschlussgeometrie **3a.** **Fig. 2d** zeigt eine Ansicht in der Ebene **A** in der die Höhe **X** des Sockels **3** dargestellt ist. Figur **2e** zeigt einen äusseren Durchmesser **11a** des länglichen Schaftes und den Durchmesser **11b** des Sockels **3.** **Fig. 2b** stellt dar den äußeren Umfang **M₁** des Mantels der Seitenwand **10** mit abgeflachter Seitenwand **10b** sowie den äußeren Umfang **M₂** des Sockels **3. d₁** entspricht der Tiefe der Fläche **7,** die der Differenz von **11b und 11a** entspricht. **d₂** entspricht der Dicke der Seitenwand **10,** die Tiefe des Bogensegmentes **6+7** kann somit eine Tiefe von **d₁ + d₂** aufweisen. Alpha **(α)** ist der Öffnungswinkel. Die **Figuren 3a** bis **3e** stellen ein Scanabutment **2** für kleine Durchmesser von bspw. 3,3 mm dar, welches nicht unter den Schutzbereich der Ansprüche fällt. In der perspektivischen Ansicht der **Figur 3a** weist das Scanabutment **2** eine planare, parallele Fläche **6** in der Durchbrechung **5a,** die in der oberen Ebene **12** des Sockels **3** (**Fig. 3b**) liegt, auf. Die Kante(n) **5c** der Durchbrechung sind vorzugsweise rechtwinklig zur Seitenwand 10 ausgebildet. Das Scanabutment weist einen Sockel **3,** insbesondere mit einer (**Fig. 2c**, **2d**) Anschlussgeometrie **3a,** insbesondere ein 6- oder 8-Kantaussenprofil, zur Befestigung des Abutments an der Stirnseite eines Implantates **1** auf. Die Anschlussgeometrie ist nicht sichtbar. Der Schaft **4,** mit Oberseite des Schaftes **4.1** bzw. obere Stirnseite und Unterseite **4.2** des Scanabutments **2** ist mit einer Durchbrechung **5a** in der Seitenwand **10** versehen in der die planare, parallele Fläche **6,** siehe **Figur 3a** und **3b****,** in der oberen Ebene **12** des Sockels **3** ausgebildet ist. In **Figur 3c** ist die Ebene **12** als Schnittebene zwischen Unterseite **4.2** des Schaftes und Oberseite **8.1** des Sockels **3,** bzw. die obere Ebene **13** auf der Oberseite **4.1** des Schaftes bzw. obere Stirnseite des Scanabutments **2** dargestellt. **Figur 3d** zeigt ein Befestigungsmittel **15,** wie Schraube, Bolzen, im Scanabutment **2** sowie die Längsseite **14** des Schaftes und dessen Höhe **Y.** Die Ausnehmung **9** im Sockel **3** kann in Form eines Loches oder Gewindes zum Durchführen bspw. einer Schraube oder eines Bolzens ausgebildet sein. Der Öffnungswinkel alpha hier 90° ist in **Figur 3e** dargestellt.

In den **Figuren 4a** bis **4d** sind erfindungsgemäße Scanabutments **2** für mittlere bis große Durchmesser bspw. 4,5 mm dargestellt. So zeigt die perspektivische Ansicht des Scanabutments **2** mit Schaft **4** und Seitenwand **10** auf einem Implantat **1** der **Figur 4a** eine Durchbrechung **5a** sowie die planare Fläche **6** und die planare, parallele Fläche **7,** die sich bildet, indem mindestens ein äußerer Durchmesser **11a** des länglichen Schaftes **4** kleiner als einer der äußeren Durchmesser **11b** des Sockels **3** ist. Die Gesamtfläche **6+7** liegt in der oberen Ebene **12** des Sockels **3** und ist vor allem im Bereich des Bogensegmentes in dem **6+7** liegen besonders gross. Figur 4b zeigt eine Draufsicht auf das Scanabutment **2** auf die Oberseite **4.1** des Schaftes sowie die Oberseite der Seitenwand **10** mit Fläche **6** und Fläche **7.** In den **Figuren 4c** und **4d** sind die Höhe **X** des Sockels **3,** die Höhe **Y** des Schaftes sowie die Längsseite 14 des Schaftes **3** des Scanabutments **2** dargestellt. Die Unterseite **8.2** des Sockels (s. auch Fig. 2c) ist auf der Stirnseite des Implantates **1.1** angeordnet.

Die **Figuren 5a** bis **5c** zeigen alternative Schaftgeometrien. Einen kreisrunden äusseren Schaft mit Durchbrechung **5a** zeigt Figur 5a, einen elliptischen Schaft mit Durchbrechung die Figur 5b und einen viereckigem Schaft die Figur 5c. Der Öffnungswinkel beträgt jeweils etwa 90°.

Die **Figuren 6a****-c** zeigen alternative Scanabutments mit ein bis drei Durchbrechungen **5a** vertikal in der Höhe entlang einer Längsachse des Schaftes verteilt. Der Öffnungswinkel beträgt in **Figur 6a** alpha = 90°, in Fig. 6b in den beiden vertikal übereinander angeordneten Durchbrechungen beträgt der Öffnungswinkel alpha ebenfalls jeweils 90°. In **Figur 6c** sind drei vertikal übereinander angeordnete Durchbrechungen mit je einem Öffnungswinkel alpha von 150° mit zusätzlicher umlaufender Fläche **7** dargestellt.

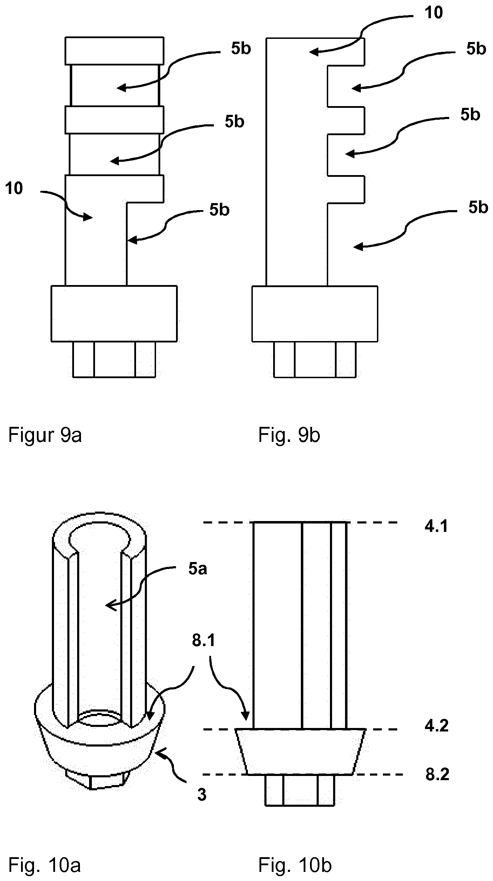

Die Scanabutments der Figuren **7a** und **7b** weisen zwei horizontal beabstandete Durchbrechungen **5a** in Form eines Rechtecks am äusseren Umfang der Seitenwand **10** auf, wobei die Seitenwand im oberen Bereich des Schaftes umlaufend **10a** ausgebildet bleibt. Die **Figuren 8a** und **8b** zeigen Scanabutments mit einer Durchbrechung **5a** der Seitenwand am äusseren Umfang in Form eines Vierecks, hier eines Trapezes **5a,** wobei in **Figur 8a** weiterhin eine umlaufende Seitenwand **10a** vorhanden ist. In **Figur 8b** reicht die trapezförmige Durchbrechung **5a** von der Unterseite des Schaftes **4.2** bis zur Oberseite **4.1** des Schaftes. In den **Figuren 9a** und **9b** sind Scanabutments **2** mit U-förmigen Vertiefungen **5b,** die entlang der Längsachse des Scanabutments weitere planare, planparallele Flächen **6** ausbilden, dargestellt, die vertikal in der Höhe versetzt liegen. Die **Figuren 10a** und **10b** zeigen ein Scanabutment **2** mit viereckiger Durchbrechung **5a,** die von der Unterseite des Schaftes **4.2** bis zur Oberseite **4.1** des Schaftes reicht und mit einem kegelstumpfartigen Sockel **3,** wobei die planare Grundfläche des Kegelstumpfes die Oberseite **8.1** des Sockels bildet. Ferner zeigen die **Figuren 10a** und **10b** die vergösserte Oberseite **8.1** und damit die vergrössterte weitere Fläche **7,** die durch die planare Grundfläche des Kegelstumpfes gebildet wird. **8.1** stellt die planare Oberseite des Sockels **3** dar und **8.2** die planare Unterseite des Sockels.

### Bezugszeichen:

- **1**: Implantat, Stirnseite (**1.1**) des Implantates (**1**)
- **2**: Scanabutment
- **3**: Sockel, insbesondere mit Anschlussgeometrie zur Befestigung des Abutments an der Stirnseite eines Implantates, **3a** Anschlussgeometrie, insbesondere 6- oder 8-Kantaussengeometrie
- **4**: Schaft, mit Oberseite des Schaftes (**4.1**) bzw. obere Stirnseite und Unterseite (**4.2**) des Schaftes
- **5a**: Durchbrechung/Unterbrechung,
- **5b**: Vertiefung
- **5c**: Kante
- **6**: planare, parallele Fläche in Durchbrechung oder Vertiefung in der oberen Ebene (**12**) des Sockels (**3**),
- **7**: planare, parallele Fläche (**7**) die sich bildet indem mindestens ein äußerer Durchmesser (**11a**) des länglichen Schaftes (**4**) kleiner als einer der äußeren Durchmesser (**11b**) des Sockels (**3**), insbesondere in der oberen Ebene (**12**) des Sockels (**3**), ist,
- **8.1**: Oberseite des Sockels (**6+7**); bzw. **8.1** obere Oberfläche des Sockels
- **8.2**: Unterseite des Sockels, bzw. **8.2** untere Oberfläche des Sockels
- **9**: Ausnehmung im Sockel, insbesondere Loch oder Gewinde zum Durchführen bspw. einer Schraube
- **10**: Seitenwand die einen Schaft bildet bzw. Seitenwand des Schaftes, **10a** umlaufende Seitenwand, **10b** abgeflachte bzw. planare Seitenwand,
- **11a**: ein äußerer Durchmesser des länglichen Schaftes
- **11b**: äußerer Durchmesser des Sockels
- **12**: Ebene (**12**) ist Schnittebene zwischen Unterseite (**4.2**) des Schaftes und Oberseite (**8.1**) des Sockels (**3**), bzw.
- **13**: obere Ebene auf der Oberseite (**4.1**) des Schaftes bzw. obere Stirnseite
- **14**: Längsseite des Schaftes
- **15**: Befestigungsmittel, wie Schraube, Bolzen

**X** = Höhe Sockel, **Y** = Höhe Schaft; **M_{1 =}äußeren Umfang (M₁** = Mantel) der Seitenwand (**10**); **M₂** = **äußeren Umfang (M₂)** des Sockels (3), **d₁** = Tiefe = Differenz von 11b und 11a, **d₂** = Dicke der Seitenwand (**10**), Öffnungswinkel (alpha, **α**)

## Patentansprüche

1. Scanabutment (2) zur Bestimmung der Lage (x,y,z-Koordinaten) der koronalen Stirnseite (1.1) eines dentalen enossalen Implantates (1), wobei das Abutment (2) einen länglichen, hohlen Schaft (4) und einen Sockel (3) an der Unterseite (4.2) des Schaftes (4) aufweist und der Sockel (3) mit zumindest einem Teil seiner Unterseite (8.2) auf der koronalen Stirnseite (1.1) des Implantates (1) anbringbar ist, wobei der Sockel (3) eine von seiner Oberseite (8.1) zur Unterseite (8.2) des Sockels (3) verlaufende Ausnehmung (9) aufweist, wobei der längliche, hohle Schaft (4) aus einer Seitenwand (10) gebildet wird,
die Seitenwand (10) zumindest teilweise im Bereich unmittelbar oberhalb des Sockels (3) an ihrem äußeren Umfang mindestens eine Durchbrechung (5a) und/oder Vertiefung (5b) in der Seitenwand (10) aufweist, wodurch durch die Durchbrechung (5a) und/oder die Vertiefung (5b) der Seitenwand (10) mindestens eine planare Fläche (6) auf der Oberseite (8.1) des Sockels (3) in der Ebene (12) zwischen Schaft (4) und Sockel (3) gebildet wird, wobei mindestens ein äußerer Durchmesser (11a) des länglichen Schaftes kleiner als einer der äußere Durchmesser (11b) des Sockels ist, wobei mindestens eine weitere, mindestens teilweise um den Schaft (4) umlaufende planare Fläche (7) in der Ebene (12) zwischen Schaft (3) und Sockel (4) gebildet wird, so dass sich eine planare Gesamtfläche als Summe aus der einen planaren Fläche und der weiteren planaren Fläche bildet, **dadurch gekennzeichnet, dass**
die Summe der Flächen von 7 bis 25 mm² beträgt.

2. Scanabutment nach Anspruch 1, **dadurch gekennzeichnet, dass** die Seitenwand (10) des länglichen, hohlen Schaftes (4)
a) zumindest teilweise entlang einer Längsseite (14) des Schaftes (4) mindestens eine Durchbrechung (5a) aufweist, und/oder
b) an ihrem äußeren Umfang mindestens zwei voneinander horizontal beabstandete Durchbrechungen (5a) aufweist, die jeweils zumindest teilweise entlang einer Längsseite (14) des Schaftes (4) verlaufen.

3. Scanabutment nach Anspruch 1 oder 2, **dadurch gekennzeichnet,**
**dass** die Seitenwand (10) des länglichen, hohlen Schaftes (4) entlang einer Längsseite (14) des Schaftes mindestens eine Durchbrechung (5a) mit einem Öffnungswinkel von 5° bis 330° im äußeren Umfang der Seitenwand gemessen von einer Längsachse des Schaftes aufweist, insbesondere weist die Seitenwand (10) eine Durchbrechung mit einem Öffnungswinkel größer gleich 90° bis 120°, vorzugsweise von 100°, plus/minus 15°, insbesondere plus/minus 10°, plus/minus 5° auf.

4. Scanabutment nach Anspruch 3, **dadurch gekennzeichnet,**
**dass** die Seitenwand (10) des länglichen, hohlen Schaftes (4) an einer Längsseite (14) des Schaftes mindestens eine Durchbrechung (5a) mit einem Öffnungswinkel von 90°, plus/minus 30° im äußeren Umfang der Seitenwand gemessen von einer Längsachse des Schaftes aufweist.

5. Scanabutment nach einem der Ansprüche 1 bis 4, **dadurch gekennzeichnet,**
**dass** die Durchbrechung (5a) der Seitenwand (10) und/oder die Vertiefung (5b) an der Seitenwand (10) des Schaftes von der Unterseite (4.2) des Schaftes (4) bis zur Oberseite (4.1) des Schaftes reichen.

6. Scanabutment nach einem der Ansprüche 1 bis 5, **dadurch gekennzeichnet,**
**dass**
a) die durch die Durchbrechung (5a) und/oder die Vertiefung (5b) der Seitenwand (10) gebildete mindestens eine planare Fläche (6) auf der Oberseite (8.1) des Sockels (3) die Form eines Bogensegmentes, Kreissegmentes, Segmentes einer Ellipse oder eines Dreiecks aufweist, oder
b) eine Durchbrechung (5a) im äußeren Umfang der Seitenwand die Form eines Vierecks, wie Rechtecks, Quadrates oder Trapezes aufweist, insbesondere reicht die Durchbrechung (5a) in der Seitenwand (10) des Schaftes (4) von der Unterseite (4.2) des Schaftes (4) bis zur Oberseite (4.1) des Schaftes (4), oder
c) mindestens eine Vertiefung (5b) in Form einer U- oder V-förmigen Nut, U- oder V-förmigen Rinne oder in Form eines Hohlzylinders vorliegt.

7. Scanabutment nach einem der Ansprüche 1 bis 6, **dadurch gekennzeichnet,**
**dass** der Sockel (3) in Form eines Kegelstumpfes vorliegt, wobei vorzugsweise die Grundfläche des Kegelstumpfes die planare Oberseite (8.1) des Sockels (3) bildet.

8. Scanabutment nach einem der Ansprüche 1 bis 7, **dadurch gekennzeichnet,**
**dass** die Höhe (X) des Sockels 0,1 bis 10 mm beträgt, insbesondere 0,75 mm bis 5 mm, bevorzugt um 1 bis 3 mm, insbesondere mit einer Toleranz von plus/minus 0,02 mm, vorzugsweise bis plus/minus 0,005 mm.

9. Scanabutment nach einem der Ansprüche 1 bis 8, **dadurch gekennzeichnet,**
**dass** die Seitenwand (10) des Schaftes die Form a) eines Zylinders, b) eines Kegels, c) eines Ellipsoids oder d) eines Polyeders bildet, und an ihrem äußeren Umfang mindestens eine Durchbrechung (5a) und/oder Vertiefung (5b) aufweist, wodurch mindestens eine planare Fläche (6) durch die Durchbrechung (5a) und/oder die Vertiefung (5b) in der Ebene (12) zwischen Schaft (4) und Sockel (3) gebildet wird, wobei die Fläche (6) parallel zur Ebene der Unterseite (8.2) des Sockels (3) und/oder der Ebene der Oberseite (4.1) des Schaftes angeordnet ist.

10. Scanabutment nach einem der Ansprüche 1 bis 9, **dadurch gekennzeichnet, dass** die Tiefe der Fläche (6) in der Ebene (12) gemessen vom äußeren Umfang (M₁) der Seitenwand (10) bis zur Längsachse des Schaftes (4) von größer gleich 0,2 bis 4 mm beträgt, insbesondere von 0,25 bis 1,5 mm, vorzugsweise von 0,3 bis 0,8 mm, besonders bevorzugt von 0,4 bis 0,7 mm.

11. Scanabutment nach einem der Ansprüche 1 bis 10, **dadurch gekennzeichnet, dass** die mindestens eine Tiefe (d₁) der weiteren Fläche (7) in der Ebene (12) gemessen vom äußeren Umfang (M₂) des Sockels (3) bis zum äußeren Umfang (M₁) des Schaftes (4) von größer gleich 0,2 bis 4 mm beträgt, insbesondere von 0,25 bis 0,8 mm, vorzugsweise von 0,3 bis 0,8 mm, besonders bevorzugt von 0,4 bis 0,7 mm.

12. Scanabutment nach einem der Ansprüche 1 bis 11, **dadurch gekennzeichnet, dass** die Dicke (d₂) der Seitenwand (10) des Schaftes (4) am äußeren Umfang des Schaftes variiert, insbesondere weist der äußere Umfang der Seitenwand (10) mindestens zwei Durchmesser auf.

13. Scanabutment nach einem der Ansprüche 1 bis 12, **dadurch gekennzeichnet, dass** das Material des Scanabutments ausgewählt ist aus einem polymeren Material, gefülltem polymeren Material, einem Metall, einem Faserverstärktem Polymer, einer Legierung, einem Hybridmaterial wie Titan, Kobaltchrom, Gold, wobei das polymere Material ausgewählt sein kann aus Fluorpolymeren, Polysulfid enthaltenden Polymeren, Polysulfon enthaltenden Polymeren, Polyaryletherketonen, Polyimiden, PAEK (Polyaryletherketonen), PC (Polycarbonat), PEEK (Polyetheretherketonen), PEK (Polyetherketonen); PEKK ( Poly(etherketonketonen)), PEEEK (Poly(etheretheretherketonen)), PEEKK (Poly(etheretherketonketonen)), PEKEKK (Poly(etherketon-etherketonketonen), PES (Polyarylsulfonen), PPSU (Polyarylsulfonen), PSU (Polysulfonen), PPS (Polyphenylensulfiden), PFA (Perfluoralkoxy-Polymeren), PFE (Poly(fluorenylen ethynylen)polymeren), PVDF (Polyvinylidenfluoriden), PCTFE (Polytetrafluoroethylen), PAI (Polyamideimiden); PI (Polyimiden), PEI (Polyetherimiden), PBI (Polybenzimidazolen).

## Claims

1. Scan abutment (2) for determination of the position (x,y,z-coordinates) of a coronal front face (1.1) of a dental endosseous implant (1), wherein the abutment (2) has a longish, hollow shaft (4) and a base (3) at the bottom side (4.2) of the shaft (4), and the base (3) with at least a part of its bottom side (8.2) is attachable onto the coronal front face (1.1) of the implant (1), wherein the base (3) has a recess (9) extending from its upper side (8.1) to the bottom side (8.2) of the base (3),
wherein the longish, hollow shaft (4) is formed from a side wall (10), wherein the side wall (10) at least in part in the region directly above the base (3) has at least one through hole (5a) and/or depression (5b) at its outer periphery in the side wall (10), thereby forming at least one planar surface (6) in the plane (12) on the upper side of the base between shaft (4) and base (3) by the through hole (5a) and/or the depression (5b), wherein at least one outer diameter (11a) of the longish shaft (4) is smaller than one of the outer diameters (11b) of the base (3), wherein at least one further planar surface (7) circumferenting the shaft (4) at least in part is formed in the plane (12) between shaft (4) and base (3), wherein the one further planar surface (7) is planar-parallel to the at least one planar surface (6), **characterised in that**,
the sum of the surfaces is from 7 to 25 mm².

2. Scan abutment according to claim 1, **characterised in that**
the side wall (10) of the longish, hollow shaft (4)
a) has at least one through hole (5a) at least in part along a longitudinal side (14) of the shaft (4), and/or
b) has at least two through holes (5a) at its outer periphery being horizontally spaced from one another, each extending at least in part along a longitudinal side (14) of the shaft (4).

3. Scan abutment according to claim 1 or 2, **characterised in that**
the side wall (10) of the longish, hollow shaft (4) has at least one through hole (5a) along a longitudinal side (14) of the shaft (4) having an opening angle of 5° to 330° in the outer periphery of the side wall, measured from a longitudinal axis of the shaft (4), in particular the side wall (10) has at least one through hole having an opening angle of 90° to 120°, in particular of 100°, plus/minus 15°, more particularly plus/minus 10°, plus/minus 5°.

4. Scan abutment according to claim 3, **characterised in that**
the side wall (10) of the longish, hollow shaft (4) has at least one through hole (5a) along a longitudinal side (14) of the shaft (4) having an opening angle of 90°, plus/minus 30°, in the outer periphery of the side wall, measured from a longitudinal axis of the shaft (4).

5. Scan abutment according to any of claims 1 to 4, **characterised in that**
the through hole (5a) of the side wall (10) and/or the depression (5b) at the side wall (10) of the shaft (4) extend from the bottom side (4.2) of the shaft (4) to an upper side (4.1) of the shaft (4).

6. Scan abutment according to any of claims 1 to 5, **characterised in that**
a) the through hole (5a) and/or depression (5b) at its outer periphery in the side wall (10) forming at least one planar surface (6) in the plane (12) on the upper side of the base in the form of an arch segment, circle segment, segment of an ellipsis or of a triangle, or
b) a through hole (5a) in the form of a quadrangle, such as rectangle, square, or trapezoid, in the outer periphery of the side wall, in particular the trough hole (5a) in the side wall (10) extends from bottom side (4.2) of the shaft (4) to the upper side (4.1) of the shaft (4), or
c) at least one depression (5b) exists in the form of a U- or V-shaped groove, U- or V-shaped channel or in the form of a hollow cylinder.

7. Scan abutment according to any of claims 1 to 6, **characterised in that**
the base (3) exists in the form of a truncated cone, wherein preferably the base of the truncated cone forming the planar upper surface (8.1) of the base (3).

8. Scan abutment according to any of claims 1 to 7, **characterised in that**
a height (X) of the base is 0.1 to 10 mm, in particular 0.75 mm to 5 mm, more preferably at about 1 to 3 mm, in particular with a tolerance of plus/minus 0.02 mm, more preferably up to plus/minus 0.005 mm.

9. Scan abutment according to any of claims 1 to 8, **characterised in that** the side wall (10) of the shaft (4) exhibits the form of a) a cylinder, b) a cone, c) an ellipsoid, d) a polyhedron, and
wherein the side wall (10) has at least one through hole (5a) and/or depression (5b) at its outer periphery, thereby forming at least one planar surface (6) in the plane (12) on the upper side of the base between shaft (4) and base (3) by the through hole (5a) and/or the depression (5b),
wherein the surface (6) is arranged parallel to the plane of the bottom side (8.2) of the base (3) and/or the plane of the upper side (4.1) of the shaft (4).

10. Scan abutment according to any of claims 1 to 9, **characterised in that**
a depth of the surface (6) in the plane (12), measured from the outer periphery (M₁) of the side wall (10) to the longitudinal axis of the shaft (4), is from greater than to equal to 0.2 to 4 mm, in particular from 0.25 to 1.5 mm, more preferably from 0.3 to 0.8 mm, most preferred from 0.4 mm to 0.7 mm.

11. Scan abutment according to any of claims 1 to 10, **characterised in that**
at least one depth (d₁) of a further surface (7) in the plane (12), measured from an outer periphery (M₂) of the base (3) to an outer periphery (M₁) of the shaft (4), is from greater than to equal to 0.2 to 4 mm, in particular from 0.25 to 0.8 mm, more preferably from 0.3 mm to 0.8 mm, most preferred from 0.4 to 0.7 mm.

12. Scan abutment according to any of claims 1 to 11, **characterised in that**
the thickness (d₂) of the side wall (10) of the shaft (4) varies at the outer periphery of the shaft, in particular the outer periphery of the side wall (10) possesses at least two diameters.

13. Scan abutment according to any of claims 1 to 12, charaterised in that the material of the scan abutment is selected from a polymeric material, filled polymeric material, a metal, a fiber-reinforced polymer, an alloy, a hybrid material, such as titanium, cobalt chrome, gold, wherein the polymeric material may be selected from fluoropolymers, polysulfide-containing polymers, polysulfone-containing polymers, polyaryletherketones, polyimides, PAEK (polyaryletherketones), PC (polycarbonate), PEEK (polyetheretherketones), PEK (polyetherketones), PEKK ( poly(etherketoneketones)), PEEEK (poly(etheretheretherketones)), PEEKK (poly(etheretherketoneketones)), PEKEKK (poly(etherketoneether-ketoneketones), PES (polyarylsulfones), PPSU (polyarylsulfones), PSU (polysulfones), PPS (polyphenylene sulfides), PFA (perfluoroalkoxy polymers), PFE (poly(fluorenylene ethynylene) polymers), PVDF (polyvinylidene fluorides), PCTFE (polytetrafluoroethylenes), PAI (polyamide imides), PI (polyimides), PEI (polyetherimides), PBI (polybenzimidazoles).

## Revendications

1. Pilier de scannage (2) pour déterminer la position (coordonnées x, y, z) de la face frontale coronale (1.1) d'un implant dentaire endo-osseux (1), le pilier (2) présentant une tige creuse allongée (4) et un socle (3) sur la face inférieure (4.2) de la tige (4) et le socle (3) pouvant être fixé par au moins une partie de sa face inférieure (8. 2) sur la face frontale coronale (1.1) de l'implant (1), le socle (3) présentant un évidement (9) s'étendant de sa face supérieure (8.1) à la face inférieure (8.2) du socle (3), la tige creuse allongée (4) étant formée par une paroi latérale (10),
la paroi latérale (10) présentant au moins partiellement, dans la zone située directement au-dessus du socle (3), sur son pourtour extérieur, au moins un percement (5a) et/ou un renfoncement (5b) dans la paroi latérale (10), ce qui fait qu'au moins une surface plane (6) est formée par le percement (5a) et/ou le renfoncement (5b) de la paroi latérale (10) sur la face supérieure (8.1) du socle (3) dans le plan (12) entre la tige (4) et le socle (3), au moins un diamètre extérieur (11a) de la tige allongée étant inférieur à l'un des diamètres extérieurs (11b) du socle, au moins une autre surface plane (7) entourant au moins partiellement la tige (4) étant formée dans le plan (12) entre la tige (3) et le socle (4), de sorte qu'une surface plane totale se forme comme somme de la première surface plane et de l'autre surface plane, **caractérisé en ce que**
la somme des surfaces est comprise entre 7 et 25 mm².

2. Pilier de scannage selon la revendication 1, **caractérisé en ce que** la paroi latérale (10) de la tige creuse allongée (4)
a) présente au moins partiellement le long d'un côté longitudinal (14) de la tige (4) au moins un percement (5a), et/ou
b) présente, sur son pourtour extérieur, au moins deux percements (5a) espacés horizontalement l'un de l'autre, s'étendant chacun au moins partiellement le long d'un côté longitudinal (14) de la tige (4).

3. Pilier de scannage selon la revendication 1 ou 2, **caractérisé en ce que**
la paroi latérale (10) de la tige creuse allongée (4) présente, le long d'un côté longitudinal (14) de la tige, au moins un percement (5a) avec un angle d'ouverture de 5° à 330° dans le pourtour extérieur de la paroi latérale mesurée à partir d'un axe longitudinal de la tige, en particulier la paroi latérale (10) présente un percement avec un angle d'ouverture supérieur ou égal à 90° à 120°, de préférence de 100°, plus ou moins 15°, en particulier plus ou moins 10°, plus ou moins 5°.

4. Pilier de scannage selon la revendication 3, **caractérisé en ce que**
la paroi latérale (10) de la tige creuse allongée (4) présente, sur un côté longitudinal (14) de la tige, au moins un percement (5a) avec un angle d'ouverture de 90°, plus ou moins 30°, dans le pourtour extérieur de la paroi latérale mesurée à partir d'un axe longitudinal de la tige.

5. Pilier de scannage selon l'une des revendications 1 à 4, **caractérisé en ce que**
le percement (5a) de la paroi latérale (10) et/ou le renfoncement (5b) sur la paroi latérale (10) de la tige s'étendent de la face inférieure (4.2) de la tige (4) à la face supérieure (4.1) de la tige.

6. Pilier de scannage selon l'une des revendications 1 à 5, **caractérisé en ce que**
a) la au moins une surface plane (6) formée par le percement (5a) et/ou le renfoncement (5b) de la paroi latérale (10), sur la face supérieure (8.1) du socle (3), présente la forme d'un segment d'arc, d'un segment de cercle, d'un segment d'ellipse ou d'un triangle, ou bien
b) un percement (5a) dans le pourtour extérieur de la paroi latérale présente la forme d'un quadrilatère, tel qu'un rectangle, un carré ou un trapèze, en particulier le percement (5a) dans la paroi latérale (10) de la tige (4) s'étend de la face inférieure (4.2) de la tige (4) jusqu'à la face supérieure (4.1) de la tige (4) ou
c) au moins un renfoncement (5b) en forme de rainure en U ou en V, en forme de canal en U ou en V ou en forme de cylindre creux est présent.

7. Pilier de scannage selon l'une des revendications 1 à 6, **caractérisé en ce que** que le socle (3) se présente sous la forme d'un cône tronqué, la surface de base du cône tronqué formant de préférence la face supérieure plane (8.1) du socle (3).

8. Pilier de scannage selon l'une des revendications 1 à 7, **caractérisé en ce que** la hauteur (X) du socle est de 0,1 à 10 mm, en particulier de 0,75 mm à 5 mm, préférablement de 1 à 3 mm, en particulier avec une tolérance de plus ou moins 0,02 mm, préférablement jusqu'à plus ou moins 0,005 mm.

9. Pilier de scannage selon l'une des revendications 1 à 8, **caractérisé en ce que**
la paroi latérale (10) de la tige constitue la forme a) d'un cylindre, b) d'un cône, c) d'un ellipsoïde ou d) d'un polyèdre, et présente, sur son pourtour extérieur, au moins un percement (5a) et/ou un renfoncement (5b), ce qui permet de former au moins une surface plane (6) par le percement (5a) et/ou le renfoncement (5b) dans le plan (12) entre la tige (4) et le socle (3), la surface (6) étant parallèle au plan de la face inférieure (8.2) du socle (3) et/ou au plan de la face supérieure (4.1) de la tige.

10. Pilier de scannage selon l'une des revendications 1 à 9, **caractérisé en ce que** la profondeur de la surface (6) dans le plan (12), mesurée depuis le pourtour extérieur (M₁) de la paroi latérale (10) jusqu'à l'axe longitudinal de la tige (4), est supérieure ou égale à 0,2 à 4 mm, en particulier de 0,25 à 1,5 mm, préférablement de 0,3 à 0,8 mm, plus préférablement de 0,4 à 0,7 mm.

11. Pilier de scannage selon l'une des revendications 1 à 10, **caractérisé en ce que** l'au moins une profondeur (d₁) de l'autre surface (7) dans le plan (12), mesurée depuis le pourtour extérieur (M₂) du socle (3) jusqu'au pourtour extérieur (M₁) de la tige (4), est supérieure ou égale à 0,2 à 4 mm, en particulier de 0,25 à 0,8 mm, préférablement de 0,3 à 0,8 mm, plus préférablement de 0,4 à 0,7 mm.

12. Pilier de scannage selon l'une des revendications 1 à 11, **caractérisé en ce que** l'épaisseur (d₂) de la paroi latérale (10) de la tige (4) varie au niveau du pourtour extérieur de la tige, en particulier le pourtour extérieur de la paroi latérale (10) présente au moins deux diamètres.

13. Pilier de scannage selon l'une des revendications 1 à 12, **caractérisé en ce que** le matériau du pilier de scannage est choisi parmi un matériau polymère, un matériau polymère chargé, un métal, un polymère renforcé par des fibres, un alliage, un matériau hybride tel que le titane, le cobalt-chrome, l'or, le matériau polymère pouvant être choisi parmi les polymères fluorés, les polymères contenant des polysulfures, les polymères contenant des polysulfones, les polyaryléthercétones, les polyimides, les PAEK (polyaryléthercétones), le PC (polycarbonate), les PEEK (polyétheréthercétones), les PEK (polyéthercétones), les PEKK ( poly(éther-cétone-cétones)), les PEEEK (poly(éther-éther-cétones)), les PEEKK (poly(éther-éther-cétone-cétones)), les PEKEKK (poly(éther-cétone-éther-cétones)), les PES (polyarylsulfones), les PPSU (polyarylsulfones), les PSU (polysulfones), les PPS (polysulfures de phénylène), les PFA (polymères perfluoroalkoxy), les PFE (polymères poly(fluorénylène éthynylène)), les PVDF (polyfluorures de vinylidène), le PCTFE (polytétrafluoroéthylène), les PAI (polyamideimides), les PI (polyimides), les PEI (polyétherimides), les PBI (Polybenzimidazoles).
